# EUROPÄISCHE PATENTSCHRIFT

(11) **EP 1 033 117 B1**
(45) Veröffentlichungstag und Bekanntmachung des Hinweises auf die Patenterteilung: **30.06.2004**
(21) Anmeldenummer: 00104098.9
(22) Anmeldetag: 28.02.2000
(51) Int. Cl.: A61F 2/38

(54) **Femurschlitten**
Femoral piece
Pièce fémorale

(30) Priorität: 02.03.1999 DE 29903766 U; 16.04.1999 DE 29906909 U
(43) Veröffentlichungstag der Anmeldung: 06.09.2000
(73) Patentinhaber: PLUS Endoprothetik AG, 6343 Rotkreuz (CH)
(72) Erfinder: Schmotzer, Hans, Dr., 5742 Kölliken (CH); Schuler, Peter, Prof. Dr. med., 76228 Karlsruhe (DE); Malzer, Udo, Dr. med., 76185 Karlsruhe (DE)
(74) Vertreter: Popp, Eugen, Dr.

(56) Entgegenhaltungen:
- EP-A- 0 707 839
- EP-A- 0 765 645

## Beschreibung

Die Erfindung betrifft einen Femurschlitten nach dem Oberbegriff des Anspruchs 1 sowie eine Femurschlitten-/ Femurgrößenlehren-Anordnung und ein Knie-Endoprothesensystem mit einem solchen Femurschlitten.

Ein derartiger Femurschlitten ist etwa aus der DE 40 41 002 C2 bekannt. Bei der zum Kniegelenkersatz mittels eines solchen Femurschlittens üblichen Operationstechnik wird von beiden Kondylen des Femurs gleich viel Knochen entfernt, so daß der anteriore bzw. ventrale Schnitt parallel zum posterioren bzw. dorsalen Schnitt ist. Bei der hierdurch definierten Position des Implantats stimmt die Implantat-Rotationsachse nicht mehr mit der durch die Lage der Seitenbänder vorgegebenen Achse überein, und die Position ist insofern nicht anatomisch, als das Implantat in der Beugung entweder auf der medialen Seite zu eng oder auf der lateralen Seite zu lose sitzt.

Die daraufhin vor einiger Zeit empfohlene Außenrotation der Schnittlehre, wobei im posterioren Bereich lateral weniger Knochen als medial, jedoch anterior-lateral mehr Knochen als auf der anterior-medialen Seiten reseziert wird, hat ebenfalls Nachteile, die durch die in DE 197 16 879 A1 der Anmelderin vorgeschlagene Ausbildung des Femurschlittens beseitigt werden sollen. Der Kern dieser Lösung liegt darin, den anterioren bzw. ventralen Schnitt in der Transversalebene zu rotieren.

Auch bei dieser neueren Lösung besteht jedoch noch Verbesserungsbedarf hinsichtlich der Optimierung der Gelenkfunktion im Zusammenwirken insbesondere mit den Seitenbändern, speziell zur Verringerung der Beanspruchung der letzteren.

Der Erfindung liegt daher die Aufgabe zugrunde, einen funktionell weiter verbesserten Femurschlitten sowie ein rationell zu fertigendes und einzusetzendes Knie-Endoprothesensystem mit einem solchen Femurschlitten als Kernstück und letztlich eine zweckmäßige Anordnung aus Femurschlitten und Femurgrößenlehre anzugeben.

Diese Aufgabe wird in ihrem ersten, tragenden Aspekt durch einen Femurschlitten mit den Merkmalen des Anspruchs 1 gelöst.

Die Erfindung schließt den wesentlichen Gedanken ein, daß es vorteilhaft ist, bei einem Kniegelenkersatz auf der Femurseite dorsal mehr Knochen zu resezieren als durch das Implantat (den Femurschlitten) wieder aufgebaut wird. Die dadurch bewirkte "Verkleinerung" des Femurschlittens im dorsalen Bereich gegenüber der ursprünglichen Dimension der (resezierten) Kondylen bzw. einem auf herkömmliche Weise angepaßten Femurschlitten führt zu einer effektiven Verringerung des Drehradius der zum Prothesensystem gehörenden Tibiaplatte und damit zu einer Entlastung der Seitenbänder.

Die zwischen den äußersten, dorsal-ventral gegenüberliegenden Punkten der Kondylenschalenflächen gemessene Verringerung der Abmessungen des Femurschlittens gegenüber der bisher üblichen Dimensionierung liegt bevorzugt im Bereich zwischen 2 und 5 %.

Dies wird durch eine solche Ausbildung der entsprechenden Femurschlitten-/Femurgrößenlehren-Anordnung erreicht, daß der Abstand eines oder mehrerer Zapfen am Femurschlitten zu dessen dorsalen Gleitflächen um 5 - 15 %, insbesondere um etwa 10 %, kleiner als der korrespondierende Abstand von Bohrungen für die Positionierung der Zapfen bei der Femurgrößenlehre zur Anlagefläche für die dorsalen Kondylenflächen des Femurs ist.

Der Abstand zwischen den dorsalen Gleitflächen und dem oder den Zapfen auf der Innenseite des Femurschlittens hat bevorzugt einen Wert im Bereich zwischen 24 und 34 mm und liegt insbesondere bei 29 mm, wobei der gewählte Betrag in einem Knie-Endoprothesensystem zur Abdeckung eines relevanten Gelenkgrößenbereiches zweckmäßigerweise konstant ist.

Der vorgeschlagene Femurschlitten zeichnet sich im übrigen dadurch aus, daß bestimmte Abmessungen zueinander in einem weitgehend konstanten Verhältnis stehen - unabhängig von der konkreten Prothesengröße. So beträgt das Verhältnis a:c zwischen der maximalen dorsal-ventralen Erstreckung und der maximalen lateralen Erstreckung des Femurschlittens etwa 0,9 ± 0,02. Die zwischen den Kondylenschalen ausgebildete Patellagrube hat, bezogen auf den am weitesten dorsal gelegenen Punkt der Kondylenschalen, bevorzugt eine Tiefe b, deren Verhältnis b:a zur maximalen dorsal-ventralen Erstreckung des Femurschlittens im Bereich zwischen 0,4 und 0,5, insbesondere bei 0,44, liegt.

Die Patellagrube ist somit nach dorsal verlängert, wodurch die Patella im gesamten funktionellen Beugebereich großflächig abgestützt werden kann.

Diese Verlängerung der Patellagrube, die im übrigen bei allen Implantatgrößen anatomisch wächst, trägt der Tatsache Rechnung, daß die patello-femurale Kontaktfläche bei herkömmlichen Femurschlitten eine relativ geringe Abstützfläche hat. In dem Bereich, in dem die Patella die Trochlea verläßt und in die fassa interkondylaris eintritt, ist nämlich bei herkömmlichen Femurkomponenten eine Abstützung nur noch im Randbereich gegeben.

Weiterhin sind bei dem vorgeschlagenen Femurschlitten die Kondylenschalen im Querschnitt (Coronalschnitt) etwas stärker verrundet als bei herkömmlichen Femurschlitten. Diese Modifikation wurde im Interesse einer verbesserten Paßfähigkeit mit dem zu einem Knie-Endoprothesensystem gehörenden speziellen Tibia-Insert vorgenommen, der aber nicht in den-Rahmen der Erfindung fällt.

Die Rückfläche des Femurschlittens trägt in einer vorteilhaften Ausführung eine in einem Vakuumplasmaverfahren erzeugte zweistufige Ti-Schicht aus einer relativ dünnen, dichten Grundschicht und einer um ein Mehrfaches dickeren, offen-porösen Deckschicht. Die dichte Grundschicht ermöglicht eine vollständige Versiegelung des beispielsweise aus CoCrMo bestehenden Femurschlittens zum Knochen hin und erhöht aufgrund des großflächigen Kontaktes mit dem Substrat die Haftfestigkeit.

Die offen-poröse und sehr rauhe Oberfläche der Deckschicht bietet ideale Bedingungen für das An- und Einwachsen von Knochensubstanz, woraus sich quasi eine "3-D-Verzahnung" ergibt, die neben Druck- und Schubkräften auch Zugkräfte übertragen kann.

Weitere Vorteile und Zweckmäßigkeiten der Erfindung sind in den Unteransprüchen bzw. der nachfolgenden Beschreibung eines bevorzugten Ausführungsbeispiels anhand der Figuren zu entnehmen. Von diesen zeigen:
- Fig. 1: eine Ansicht (von proxima) auf einen Femurschlitten gemäß einer Ausführungsform der Erfindung,
- Fig. 2: den Femurschlitten gemäß Fig. 1 in einer medialen Schnittdarstellung (Sagittalschnitt),
- Fig. 3: eine Draufsicht auf eine Ausführungsform einer Femurgrößenlehre,
- Fig. 4: eine Seitenansicht derselben,
- Fig. 5a, 5b: eine Gegenüberstellung einer herkömmlichen mit einer hier zur Ausführung vorgeschlagenen Anordnung eines Femurschlittens an einem Femur und
- Fig. 6a, 6b: rasterelektronenmikroskopische Querschnittsdarstellungen eines herkömmlichen Schichtaufbaus bzw. einer Ausführungsform des hier vorgeschlagenen Schichtaufbaus der Rückflächenbeschichtung eines Femurschlittens.

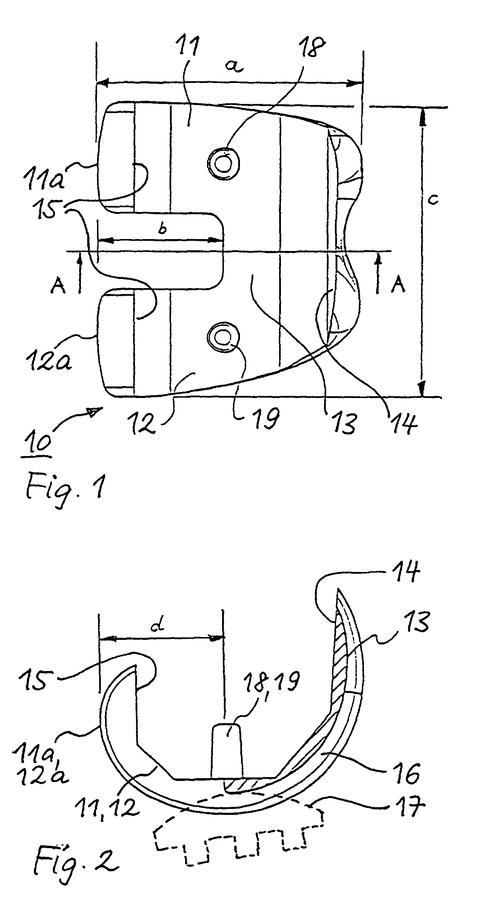

In Fig. 1 und 2 ist die üblicherweise als Femurschlitten bezeichnete Femurkomponente 10 einer Knie-Endoprothese dargestellt. Der Femurschlitten 10 umfaßt zwei konvex gewölbte Kondylenschalen 11, 12 und ein Patellaschild 13, welches die beiden Kondylenschalen 11, 12 starr miteinander verbindet.

Die Kondylenschalen 11, 12 und das Patellaschild 13 definieren innenseitig anteriore und posteriore Paßflächen 14, 15, die einem femuralen Ventral- bzw. Dorsalschnitt entsprechen und einer ventralen und dorsalen Sägeschnittfläche am distalen Ende des für die Anpassung des Femurschlittens bearbeiteten Femurs zugeordnet sind. Die konvexe Außengestaltung der Kondylenschalen 11, 12 bestimmt im posterioren Bereich dorsale Gleitflächen 11a, 12a, auf denen bei einer Beugung der Knie-Endoprothese die korrespondierenden Flächen des Tibia-Inserts gleiten. Das gegenüber den konvexen Außenflächen der Kondylenschalen 11, 12 zurückspringende Patellaschild 13 definiert eine sogenannte Patellagrube 16, in der eine - in Fig. 2 gestrichelt angedeutete, nicht zum Femurschlitten 10 gehörende - Patellakomponente 17 der Knie-Endoprothese abgestützt ist.

Zur Verankerung und zentralen Führung des Femurschlittens 10 am Femur sind auf der Innenseite des Femurschlittens zwei Zapfen 18, 19 ausgebildet, deren Längsachse im wesentlichen parallel zur posterioren Paßfläche 15 ausgerichtet ist. Diese Zapfen greifen in mit Hilfe einer entsprechenden Bohrlehre (siehe dazu weiter unten) korrespondierend positionierte Bohrlöcher im Femur ein und geben dem Femurschlitten, neben den Paßflächen, zusätzlich Halt am Knochen.

Die Sicherstellung einer funktionell optimierten Ersatzfunktion für zerstörte Gleitflächen am Femur durch den Femurschlitten erfordert einerseits eine möglichst genaue Konstruktion der anatomischen Verhältnisse und Abmessungen, schließt aber im Rahmen der Erfindung auch eine spezifische Modifikation ein, wie nachfolgend erläutert wird.

Eine der relevanten Dimensionen des Femurschlitten 10 ist die maximale anterior-posteriore bzw. dorsal-ventrale Erstreckung der Kondylenschalen 11, 12, die in Fig. 1 mit a bezeichnet ist. Eine weitere relevante Dimension ist die maximale laterale Erstreckung des Femurschlittens, d. h. der Abstand zwischen dem lateralsten Punkt der lateralen Kondylenschale 11 und dem am weitesten medial gelegenen Punkt der medialen Kondylenschale 12, der in Fig. 1 mit c bezeichnet ist. Weiterhin von Bedeutung ist der Abstand zwischen dem äußersten posterioren Punkt der dorsalen Gleitflächen 11a, 12a der Kondylenschalen 11, 12 bis zur posterioren Begrenzungskante des Patellaschildes 13, der in Fig. 1 mit b bezeichnet ist. Schließlich ist bedeutsam der Abstand zwischen den äußersten posterioren Punkten der dorsalen Gleitflächen 11a, 12a und der Längsachse der (in ein und derselben Coronalebene liegenden) Zapfen 18, 19, der in Fig. 2 mit d bezeichnet ist. Beim beschriebenen Ausführungsbeispiel beträgt das Verhältnis a:c 0,9 und das Verhältnis b:a 0,44. Aus biomechanischen und operationstechnischen Gründen hat es sich als sinnvoll erwiesen, die Größe d (Gleitflächen-Zapfen-Abstand) für alle Femurschlittengrößen eines Knie-Endoprothesensystems auf einen einheitlichen Wert einzustellen. Im vorliegenden Fall beträgt dieser 29 mm.

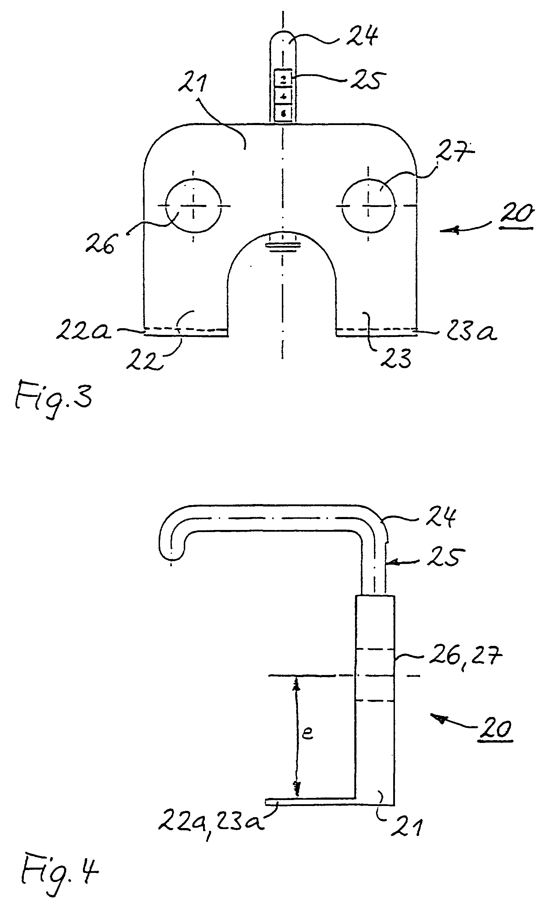

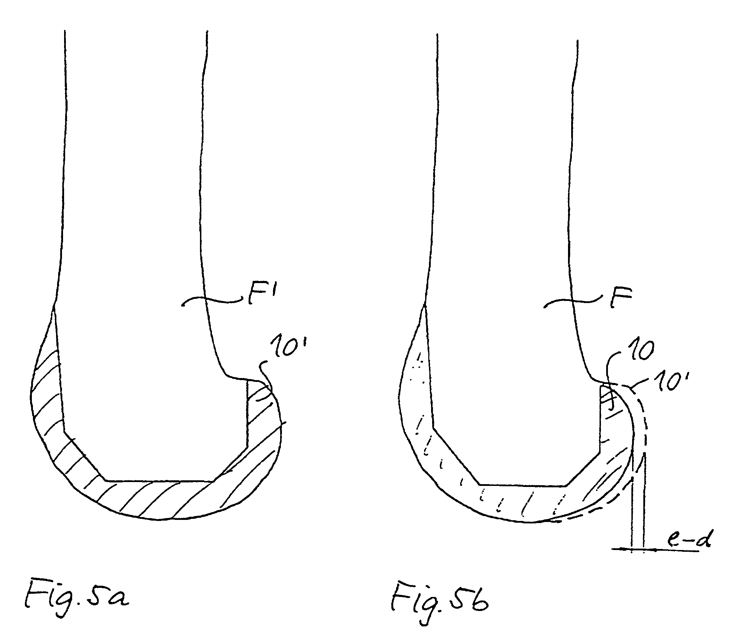

Zur Bestimmung der korrekten Femurschlittengröße dient eine in Fig. 3 und 4 gezeigte Femurgrößenlehre 20. Diese umfaßt ein Grundteil 21, das zwei Flanken 22 und 23 mit jeweils einem rechtwinklig abgewinkelten Anlageabschnitt 22a, 23a zur Anlage an die Kondylen eines mit einem Femurschlitten (Fig. 1 und 2) zu versehenden Oberschenkelknochens aufweist.

Im Grundteil 21 ist mittig eine zweifach abgewinkelte Meßzunge 24 in zur Erstreckungsebene der Anlageabschnitte 22a, 23a senkrechter Richtung verschieblich geführt. Die Meßzunge 24 trägt eine Skaleneinteilung 25, die die maximale anterior-posteriore Erstreckung des Femurkopfes, d. h. der Kondylen, und damit dem Arzt die benötigte Implantatgröße angibt. Im Grundteil 21 der Femurgrößenlehre 20 sind zwei Zapfenloch-Bohrungen 26, 27 vorgesehen, die - gemäß einer Bohrlehren-Zusatzfunktion der Femurgrößenlehre - zur Positionierung von Zapfenlöchern entsprechend den Zapfen 18, 19 des Femurschlittens 10 nach Fig. 1 im Femur dienen. Die Achsen der Zapfenloch-Bohrungen 26, 27 haben einen Abstand e zu den Anlageflächen der Anlageabschnitte 22a, 23a. Dieser Abstand stellt - neben dem Gleitflächen-Zapfen-Abstand d beim Femurschlitten 10 selbst (vgl. Fig. 2) - aus dem folgenden Grund eine zusätzliche relevante Dimension bei der konkreten Realisierung einer Knie-Endoprothese dar:

Um die erwähnten Zapfenlöcher, die im übrigen neben der Positionierung des Implantats auch zur Positionierung der Schnittlehren zur Erzeugung der verschiedenen Sägeschnitte am Femur dienen, in den Knochen zu bohren, werden in die Zapfenloch-Bohrungen 26, 27 Bohrbüchsen (nicht dargestellt) gesteckt.

Es hat sich - insbesondere unter dem Blickwinkel einer verringerten Beanspruchung der Seitenbänder bei einer Flexion des künstlichen Kniegelenkes - als vorteilhaft herausgestellt, dorsal am Femur mehr Knochen zu resezieren, als dort durch die Dicke der dorsalen Abschnitte der Kondylenschalen wieder aufgebaut wird. Aus diesem Grund wird der Abstand e größer gewählt, als der korrespondierende Abstand d (Fig. 2). In der bevorzugten Ausführungsform liegt die relative Abstandsverringerung, d. h. die Größe (e - d)/d, bei etwa 10 %.

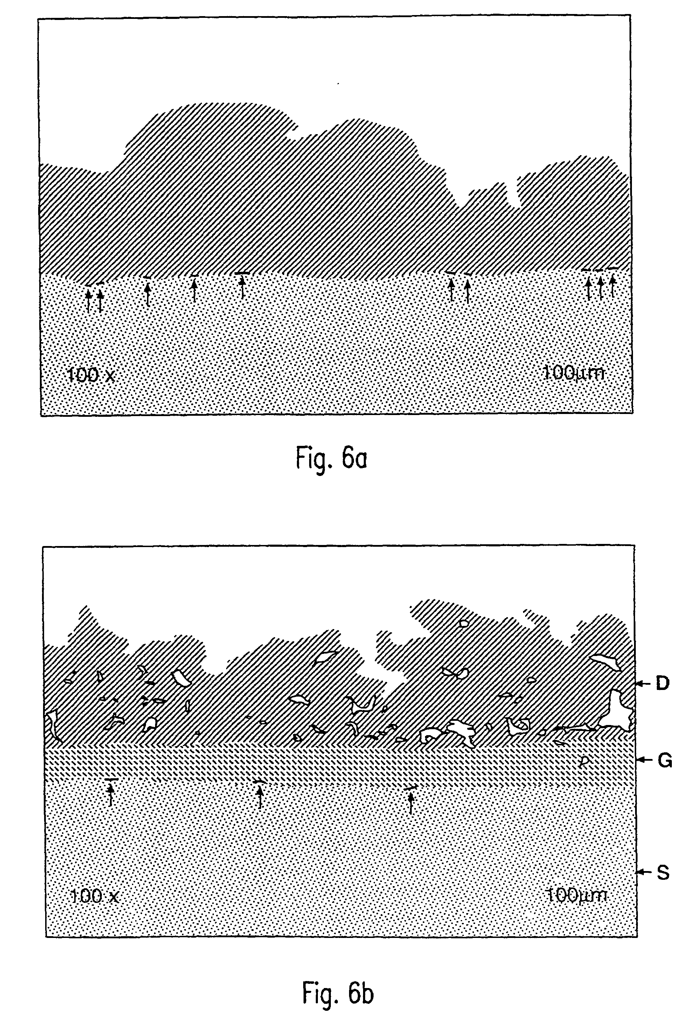

Der erreichte Effekt ist in der skizzenhaften Gegenüberstellung der herkömmlichen Art der Anbringung eines Femurschlittens 10' an einem Femur F' in Fig. 5a mit einer Ausführung der hier vorgeschlagenen Anordnung in Fig. 5b zu erkennen. Die anterior-posteriore Erstreckung des Femurschlittens 10 in Fig. 5b an einem im dorsalen Bereich weiter resezierten Femur F ist um den Betrag (e - d) kleiner als beim herkömmlichen Implantat 10'.

Da der Abstand e an der Femurgrößenlehre, die ja für alle Implantatgrößen gleichermaßen eingesetzt wird, fest vorgegeben ist und nach obigem auch bei der Ausführung des Femurschlittens der Abstand d bevorzugt für alle Implantatgrößen konstant gehalten wird, ergeben sich für die verschiedenen Implantatgrößen geringfügig unterschiedliche geometrische Verhältnisse. Dies ist aber mit Blick auf die herstellungs- und handhabungstechnischen Vorteile eines solchen Systems akzeptabel.

Fig. 6b zeigt - in Gegenüberstellung zu einer herkömmlichen Femurschlitten-Beschichtung in Fig. 6a - in einer rasterelektronenmikroskopischen Querschnittsdarstellung einen zweikomponentigen Titan-Beschichtungsaufbau aus einer etwa 50µm starken, dichten Grundschicht G und einer im Mittel annähernd 250µm dicken, offen-porösen Deckschicht D auf einem CoCrMo-Substrat S. Die Dicke und mittlere Rauheit der durch Vakuumplasmabeschichtung erzeugten Beschichtung gemäß Fig. 6b ist zwar vergleichbar mit der bekannten, gespritzten Beschichtung nach Fig. 6a, hervorzuheben ist dieser gegenüber jedoch die offen-poröse Struktur und die wesentlich verringerte Anzahl von (in beiden Figuren durch vertikale Pfeile gekennzeichneten) Grenzflächendefekten.

Die Ausführung der Erfindung ist nicht auf das oben beschriebene Ausführungsbeispiel beschränkt, sondern auch in Abwandlungen möglich, die insbesondere Abweichungen von den konkreten Maß- und Verhältniswertangaben einschließen und dabei in den Bereich der Ansprüche fallen.

### Bezugszeichenliste

- 10, 10': Femurschlitten
- 11, 12: Kondylenschalen
- 11a, 12a: dorsale Gleitflächen
- 13: Patellaschild
- 14: anteriore Paßfläche
- 15: posteriore Paßfläche
- 16: Patellagrube
- 17: Patellakomponente
- 18, 19: Zapfen
- 20: Femurgrößenlehre
- 21: Grundteil
- 22, 23: Flanken
- 22a, 23a: Anlageabschnitte
- 24: Meßzunge
- 25: Skaleneinteilung
- 26, 27: Zapfenloch-Bohrungen

- a, b, c , d, e: Abstände
- A-A: Schnittebene
- D: Deckschicht
- F, F': Femur (bearbeitet)
- G: Grundschicht
- S: Substrat

## Patentansprüche

1. Femurschlitten (10), mit zwei konvex gewölbten Kondylenschalen (11, 12), die anterior durch ein Patellaschild (13) starr miteinander verbunden sind, wobei die Kondylenschalen außenseitig dorsale Femurschlitten-Gleitflächen (11a, 12a) definieren,
**dadurch gekennzeichnet,**
**daß** die Gesamterstreckung des Femurschlittens in anteriorposteriorer Richtung kleiner als die entsprechende ursprüngliche Gesamterstreckung der Kondylen eines Femurs (F) ist, an den der Femurschlitten angepaßt ist.

2. Femurschlitten nach Anspruch 1,
**dadurch gekennzeichnet,**
**daß** die Gesamterstreckung des Femurschlittens um 2-5 % kleiner ist als die Gesamterstreckung der Kondylen.

3. Femurschlitten nach Anspruch 1 oder 2,
**dadurch gekennzeichnet,**
**daß** der Femurschlitten (10) innenseitig mindestens einen, insbesondere zwei Zapfen (18, 19) trägt und zwischen der Längsachse des bzw. der Zapfen und den von dieser am weitesten entfernten Punkten der dorsalen Gleitflächen (11a, 12a) ein Gleitflächen-Zapfen-Abstand (d) definiert ist, und
**daß** der Gleitflächen-Zapfen-Abstand im Bereich zwischen 24 und 34 mm liegt, insbesondere 29 mm beträgt.

4. Femurschlitten nach einem der vorangehenden Ansprüche,
**dadurch gekennzeichnet,**
**daß** das Verhältnis (a:c) der maximalen anterior-posterioren Erstreckung (a) zur maximalen lateralen Erstreckung (c) des Femurschlittens (10) im wesentlichen 0,9 beträgt.

5. Femurschlitten nach einem der vorangehenden Ansprüche,
**dadurch gekennzeichnet,**
**daß** eine zwischen den Kondylenschalen (11, 12) ausgebildetete Patellagrube (16), bezogen auf den entferntesten dorsalen Punkt der Kondylenschalen, eine Tiefe (b) hat, deren Verhältnis (b:a) zur maximalen anterioren-posterioren Erstreckung des Femurschlittens (10) im Bereich zwischen 0,4 und 0,49 liegt, insbesondere 0,44 beträgt.

6. Femurschlitten nach einem der vorangehenden Ansprüche,
**dadurch gekennzeichnet,**
**daß** innenseitig eine durch Plasmavakuumbeschichtung gebildete zweikomponentige Beschichtung (G, D) aus einer dünneren, dichten Grundschicht (G) und einer dickeren, offen-porösen Deckschicht (D) im wesentlichen aus Titan vorgesehen ist.

7. Knie-Endoprothesensystem mit einer Mehrzahl von Femurschlitten (10) unterschiedlicher Größe nach einem der vorangehenden Ansprüche,
**dadurch gekennzeichnet,**
**daß** der Betrag des Gleitflächen-Zapfen-Abstandes (d) für alle Femurschlitten des Knie-Endoprothesensystems gleich ist.

8. Femurschlitten-/Femurgrößenlehren-Anordnung, umfassend mindestens einen Femurschlitten (10) nach einem der Ansprüche 3-6 und
eine Femurgrößenlehre (20), welche ein Grundteil (21) mit ebenen Anlageabschnitten (22a, 23a) zur Anlage an die dorsalen Kondylenflächen eines Femurs (F) und einer rechtwinklig auf diesen Anlageflächen stehende Bohrlehrenfläche hat, die mindestens eine Zapfenloch-Bohrung (26, 27) zur Festlegung mindestens einer Bohrung im Femur zur Aufnahme mindestens eines Zapfens (18, 19) des Femurschlittens (10) trägt,
wobei zwischen dem Mittelpunkt der Zapfenloch-Bohrung bzw. Zapfenloch-Bohrungen und der Kondylenanlagefläche der Anlageabschnitte (22a, 23a) ein Bohrungs-Gleitflächen-Abstand (e) definiert ist, der größer als der Gleitflächen-Zapfen-Abstand (d) am Femurschlitten ist.

9. Femurschlitten-/Femurgrößenlehren-Anordnung nach Anspruch 8,
**dadurch gekennzeichnet,**
**daß** der Bohrungs-Gleitflächen-Abstand (e) um 5-15 %, insbesondere um 10 %, größer als der Gleitflächen-Zapfen-Abstand (d) ist.

## Claims

1. A femoral component (10) comprising two convexly curved condylar shells (11, 12) rigidly connected to one another in the anterior direction by a patellar shield (13), wherein the outer surfaces of the condylar shells define dorsal sliding surfaces (11a, 12a) of the femoral component, **characterised in that** the overall extent of the femoral component in the anterior-posterior direction is smaller than the corresponding original overall extent of the condyles of a femur (F) to which the femoral component is adapted.

2. A femoral component according to claim 1, **characterised in that** the overall extent of the femoral component is smaller by 2%-5% than the overall extent of the condyles.

3. A femoral component according to claim 1 or 2, **characterised in that** the femoral component (10) carries at least one, in particular two pegs (18, 19) on its inner surface, and a sliding surface - peg distance (d) is defined between the longitudinal axis of the peg or pegs and the points of the dorsal sliding surfaces (11a, 12a) furthest away therefrom, and **in that** the sliding surface - peg distance lies in the range between 24 mm and 34 mm, in particular is 29 mm.

4. A femoral component according to any one of the preceding claims, **characterised in that** the ratio (a:c) of the maximum anterior-posterior extent (a) to the maximum lateral extent (c) of the femoral component (10) is substantially 0.9.

5. A femoral component according to any one of the preceding claims, **characterised in that** a patellar groove (16) formed between the condylar shells (11, 12), measured to the furthest dorsal point of the condylar shells, has a depth (b), the ratio (b:a) of which to the maximum anterior-posterior extent of the femoral component (10) lies in the range between 0.4 and 0.49, in particular is 0.44.

6. A femoral component according to any one of the preceding claims, **characterised in that** a two-component coating (G, D) formed by vacuum plasma coating is provided on its inner surface and comprises a thinner, impervious base layer (G) and a thicker, open-porous covering layer (D) substantially of titanium.

7. A knee endoprosthesis system comprising a plurality of femoral components (10) of different sizes according to any one of the preceding claims, **characterised in that** the sliding surface - peg distance (d) is the same for all femoral components of the knee endoprosthesis system.

8. A femoral component / femoral gauge arrangement comprising at least one femoral component (10) according to any one of claims 3-6 and a femoral gauge (20) having a base part (21) with flat contact portions (22a, 23a) for resting against the dorsal condylar surfaces of a femur (F) and with a drilling gauge surface lying at right angles to these contact surfaces and provided with at least one peg-hole bore (26, 27) for determining the position of at least one bore in the femur for receiving at least one peg (18, 19) of the femoral component (10), wherein, between the centre point of the peg-hole bore or bores and the condylar contact surface of the contact portions (22a, 23a), a bore - sliding surface distance (e) is defined which is greater than the sliding surface - peg distance (d) on the femoral component.

9. A femoral component / femoral gauge arrangement according to claim 8, **characterised in that** the bore - sliding surface distance (e) is greater by 5%-15%, in particular 10%, than the sliding surface - peg distance (d).

## Revendications

1. Bouclier fémoral (10), comportant deux coques condylaires (11, 12) à courbure convexe, qui sont reliées l'une à l'autre de manière rigide dans la zone antérieure par une plaque patellaire (13), les coques condylaires étant délimitées du côté extérieur par des surfaces de glissement dorsales (11a, 12a) du bouclier fémoral, **caractérisé en ce que** l'extension totale du bouclier fémoral dans la direction antérieure-postérieure est inférieure à l'extension totale initiale des condyles d'un fémur (F), auquel est adapté le bouclier fémoral.

2. Bouclier fémoral selon la revendication 1, **caractérisé en ce que** l'extension totale du bouclier fémoral est de 2 à 5 % inférieure à l'extension totale des condyles.

3. Bouclier fémoral selon la revendication 1 ou 2, **caractérisé en ce que** le bouclier fémoral (10) porte du côté intérieur au moins un, de préférence deux tenons (18, 19), et une distance (d) entre surfaces de glissement et tenon est délimitée entre l'axe longitudinal du ou des tenon(s) et les points les plus éloignés de ceux-ci des surfaces de glissement dorsales (11a, 12a), et **en ce que** la distance (d) entre surfaces de glissement et tenon se situe dans une plage entre 24 et 34 mm, en particulier 29 mm.

4. Bouclier fémoral selon l'une quelconque des revendications précédentes, **caractérisé en ce que** le rapport (a:c) entre l'extension antérieure-postérieure (a) maximale et l'extension latérale (c) du bouclier fémoral (10) est sensiblement égal à 0,9.

5. Bouclier fémoral selon l'une quelconque des revendications précédentes, **caractérisé en ce qu'**une cavité patellaire (16), réalisée entre les coques condylaires (11, 12), présente par rapport au point dorsal des coques condylaires le plus éloigné une profondeur (b) dont le rapport (b:a) avec l'extension antérieure-postérieure (a) maximale du bouclier fémoral (10) se situe dans une plage entre 0,4 et 0,49, en particulier 0,44.

6. Bouclier fémoral selon l'une quelconque des revendications précédentes, **caractérisé en ce qu'**il est prévu sur la face intérieure un revêtement (G, D) à deux composants, déposé par un procédé plasma sous vide, formé par une couche de base (G) dense, plus mince et une couche de couverture (D) à pores ouverts plus épaisse essentiellement en titane.

7. Système d'endoprothèse du genou comprenant une pluralité de boucliers fémoraux (10) de différentes dimensions selon l'une quelconque des revendications précédentes, **caractérisé en ce que** la valeur de la distance (d) entre surfaces de glissement et tenon est identique pour toutes les boucliers fémoraux du système d'endoprothèse du genou.

8. Système de bouclier fémoral et gabarit fémoral, comprenant au moins un bouclier fémoral (10) selon l'une quelconque des revendications 3 à 6 et un gabarit fémoral (20), qui comporte une partie de base (21) avec des tronçons d'appui (22a, 23a) plans, destinés à venir en appui contre les surfaces condylaires dorsales d'un fémur (F), et une face de gabarit de perçage, qui est disposée perpendiculairement sur ces surfaces d'appui et qui comporte au moins un trou pour tenon (26, 27) pour définir dans le fémur au moins un trou destiné à recevoir au moins un tenon (18, 19) du bouclier fémoral (10), dans lequel système une distance (e) entre trou et surfaces de glissement est définie entre le centre du trou pour tenon ou des trous pour tenons et la face d'appui condylaire des tronçons d'appui (22a, 23a), laquelle distance est supérieure à la distance (d) entre surfaces de glissement et tenon sur le bouclier fémoral.

9. Système de bouclier fémoral et gabarit fémoral selon la revendication 8, **caractérisé en ce que** la distance (e) entre trou et surfaces de glissement est supérieure de 5 à 15 %, en particulier de 10 %, à la distance (d) entre surfaces de glissement et tenon.
